# EUROPÄISCHE PATENTSCHRIFT

(11) **EP 0 909 553 B1**
(45) Veröffentlichungstag und Bekanntmachung des Hinweises auf die Patenterteilung: **14.01.2004**
(21) Anmeldenummer: 98118955.8
(22) Anmeldetag: 07.10.1998
(51) Int. Cl.: A61F 2/06, B23K 26/00

(54) **Verfahren zur Herstellung feinstrukturierter medizintechnischer Implantate**
Process for manufacturing finely patterned medical implants
Procédé de fabrication d'implants médicaux finement structurés

(30) Priorität: 14.10.1997 DE 19745294
(43) Veröffentlichungstag der Anmeldung: 21.04.1999
(73) Patentinhaber: BIOTRONIK Mess- und Therapiegeräte GmbH & Co Ingenieurbüro Berlin, 12359 Berlin (DE)
(72) Erfinder: Momma, Carsten, Dr., 30171 Hannover (DE); Nolte, Stefan, 30459 Hannover (DE); von Alvensleben, Ferdinand, Dr.-Ing., 33181 Bad Wünnenberg (DE); Bolz, Armin, Dr., 91054 Buckenhof (DE)
(74) Vertreter: Hübner, Gerd, Dipl.-Phys.

(56) Entgegenhaltungen:
- EP-A- 0 714 641
- EP-A- 0 842 729
- WO-A-95/27587
- NOLTE S ET AL: "Micromachining with femtosecond lasers" PROCEEDINGS OF THE 1998 CONFERENCE ON LASERS AND ELECTRO-OPTICS, CLEO;SAN FRANCISCO, CA, USA MAY 3-8 1998,1998, Seiten 510-511, XP002092164 Conf Lasers Electro Opt Eur Tech Dig;Conference on Lasers and Electro-Optics Europe - Technical Digest 1998 IEEE, Piscataway, NJ, USA

## Beschreibung

Die Erfindung betrifft ein Verfahren zur Herstellung feinstrukturierter medizintechnischer Implantate durch Laser-Materialbearbeitung und insbesondere ein Laser-Schneiden von bioresorbierbaren Gefäßwandstützen.

Zum Hintergrund der Erfindung ist festzuhalten, daß in der Medizintechnik zahlreiche Implantate bekannt sind, die aus medizintechnischen Werkstoffen, wie Metall-Legierungen oder Polymeren bestehen und mittels Schneidverfahren strukturiert werden. Als Beispiel sind Gefäßwandstützen etwa zum Einsatz im Herzen zu nennen, die im Fachjargon auch als "Stents" bezeichnet werden. Um das mit dem Implantat in Kontakt kommende Gewebe, wie z. B. die Innenwand einer Herzader bei einem intravaskulären "Stent", nicht unnötig zu irritieren, sollen die Strukturkanten des Implantats möglichst glatt und gratfrei sein.

Im Stand der Technik werden feinstrukturierte medizintechnische Implantate durch aus der herkömmlichen Materialbearbeitungstechnik stammende Laser-Schneidverfahren hergestellt. Grundsätzlich erlaubt die Verwendung von Lasern dabei die Erzeugung kleinster Strukturen mit hoher Präzision bei hoher Bearbeitungsgeschwindigkeit.

Die angesprochenen herkömmlichen Laser-Schneidverfahren arbeiten in der Regel mit Dauerstrichlasern oder allenfalls gepulsten Lasern mit Pulsdauern in der Größenordnung von Nanosekunden bis Millisekunden. Damit ist die Einwirkdauer des Lasers auf den Werkstoff an der jeweiligen Bearbeitungsstelle so groß, daß neben dem eigentlichen Durchschneiden des Werkstoffes eine beträchtliche Wärmeentwicklung in der mikroskopischen Umgebung der Schnittkante hervorgerufen wird. Dadurch werden kleinste Mengen des Werkstoffes geschmolzen, die nach der Einwirkdauer des Laserstrahles oder zwischen den einzelnen langen Pulsen ungeordnet erstarren. Dies bewirkt die nachteilige Vergratung der Schnittkanten.

Die vorstehend erörterte Vergratung macht Nachbearbeitungsschritte zur Entgratung notwendig. Ein übliches Verfahren ist hierfür das Elektropolieren, was jedoch in der Regel sehr aufwendig ist und nur geringfügige Verbesserungen schafft. Ferner ist das Elektropolieren wegen der geringen elektrischen Leitfähigkeit vieler Werkstoffe, wie sie insbesondere bei Polymeren vorliegt, nicht einsetzbar.

Zur Vermeidung der vorstehenden Nachteile kann auch an ein alternatives Schneidverfahren gedacht werden, wie z. B. die Funkenerosion. Diese wiederum bringt einen höheren Zeitaufwand bei der Materialbearbeitung sowie eine geringere Präzision mit sich. Auch ist aufgrund der geringeren elektrischen Leitfähigkeit von Polymeren der Einsatz der Funkenerosion bei diesen Werkstoffen nicht möglich.

Schließlich ist darauf hinzuweisen, daß aus der WO 95/27587 A ein Verfahren zur Laser-Materialbearbeitung von Werkstoffen bekannt ist, bei dem mit sehr kurzen Laserpulsen im Femtosekunden-Bereich gearbeitet wird. Dabei werden verschiedene Proben, wie eine Goldprobe, eine dünne Glasplatte und eine Körpergewebe-Probe (Hornhaut des Auges) mit kurzen Laserpulsen im Femtosekunden-Bereich bestrahlt und dadurch mikroskopische Öffnungen und Ablationsbereiche geschaffen.

Ausgehend von der geschilderten Problematik liegt der Erfindung die Aufgabe zugrunde, ein Herstellungsverfahren für feinstrukturierte medizintechnische Implantate durch Laser-Materialbearbeitung anzugeben, mit dem ohne aufwendige Nachbehandlungsschritte möglichst gratfreie Bearbeitungskanten erzeugt werden.

Diese Aufgabe wird durch die im Anspruch 1 bzw.2 angegebenen Merkmale gelöst. Demnach wird bei der Bearbeitung mit einem gepulsten Laserstrahl einer Pulsdauer im Bereich von 10 bis 10000 Femtosekunden und einer so abgestimmten Laser-Einstrahlung gearbeitet, daß eine schmelzfreie, materialschonende Bearbeitung des Werkstoffes stattfinden kann. Unter "schmelzfrei" ist dabei zu verstehen, daß unkontrollierte Schmelzprozesse im zu bearbeitenden Werkstoff nicht mehr auftreten, wodurch eine Vergratung von Bearbeitungs- und insbesondere Schnittkanten vermieden wird. Die Parameter Pulswiederhol-Frequenz und/oder Laser-Leistung und/oder die Verfahrgeschwindigkeit des Werkstückes relativ zum Laserstrahl sind in ihrer Kombination entsprechend anzupassen. Ein derartiges Herstellungsvergahren ist bereits in der EP-A-0 842 729 (Stand der Technik gemäß Art. 54(3) und (4) EPÜ) angegeben. Die Vermeidung unkontrollierter Schmelzprozesse wird generell durch die Verkürzung der Laserpulsdauer bis in den angegebenen Bereich möglich, was zu einer Verminderung der benötigten Laserpulsenergie führt und die Wärmeentwicklung so drastisch reduziert, daß die unerwünschten unkontrollierten Schmelzprozesse unterbleiben. Damit werden bei einem Schneiden von medizintechnischen Werkstoffen die Vergratung der Schnittkanten und eine thermische bzw. photochemische Beeinträchtigung des Werkstück-Materials verhindert. Letzteres ist unter den Begriff "materialschonend" zu subsumieren. Gemäß den Ansprüchen 1 bzw.2 kann ein Laser-Scanner zur Führung des Laserstrahls über des Werkstück eingesetzt werden bzw. das Werkstück durch schneidende Laser-bearbeitung mit einer Struktur aus miteinander Vernekten Stegen versehen werden.

Zum Hintergrund der erfindungsgemäß eingesetzten Femtosekunden-Lasertechnik ist kurz festzuhalten, daß derartig kurze Pulsdauem in größerem Maßstab bisher lediglich in der Daten- und Informationstechnik eingesetzt wurden, wo aufgrund der kurzen Pulsdauern hohe Datenübertragungsraten erreicht werden konnten. Entsprechend der dortigen Anwendung sind allerdings nur niedrige Laser-Leistungen erforderlich.

Bevorzugte Ausgestaltungen des erfindungsgemäßen Verfahrens sowie weitere Merkmale, Einzelheiten und Vorteile der Erfindung sind den Unteransprüchen und der nachfolgenden Beschreibung entnehmbar, in der ein Ausführungsbeispiel des Erfindungsgegenstandes anhand der beigefügten Zeichnungen näher erläutert wird. Es zeigen:
- Fig. 1: eine Abwicklung der Struktur eines "Stents" und
- Fig. 2: eine schematische Darstellung einer Vorrichtung zum ultra-kurz gepulsten Laser-Schneiden des "Stents" gemäß Fig. 1.

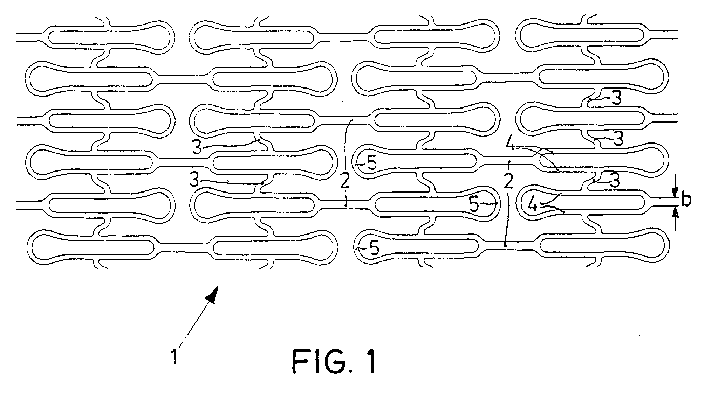

Wie aus Fig. 1 deutlich wird, besteht ein an sich bekannter "Stent" 1 aus einem feinstrukturierten Netz von Längsstegen 2 und diese verbindenden Querstegen 3. Die Längsstege 2 verzweigen sich dabei in einander parallele Stränge 4, die jeweils am Ende über einen Bogen 5 paarweise miteinander verbunden sind. Links und rechts bezogen auf Fig. 1 setzen sich die Längsstege 2 mit ihren Verzweigungssträngen 4 bis zum Ende des rohrförmigen "Stents" fort. In Richtung der Querstege 3 ist die Struktur zylindrisch gebogen, so daß die oben bezüglich Fig. 1 auslaufenden Querstege 3 in die unten auslaufenden Querstege 3 übergehen. Hinsichtlich der Größenordnung liegen die Breiten b der Stege 2, 3 im Submillimeterbereich.

Der in Fig. 1 gezeigte "Stent" ist bioresorbierbar. Er besteht aus dem Material Poly-Hydroxy-Buttersäure (PHB).

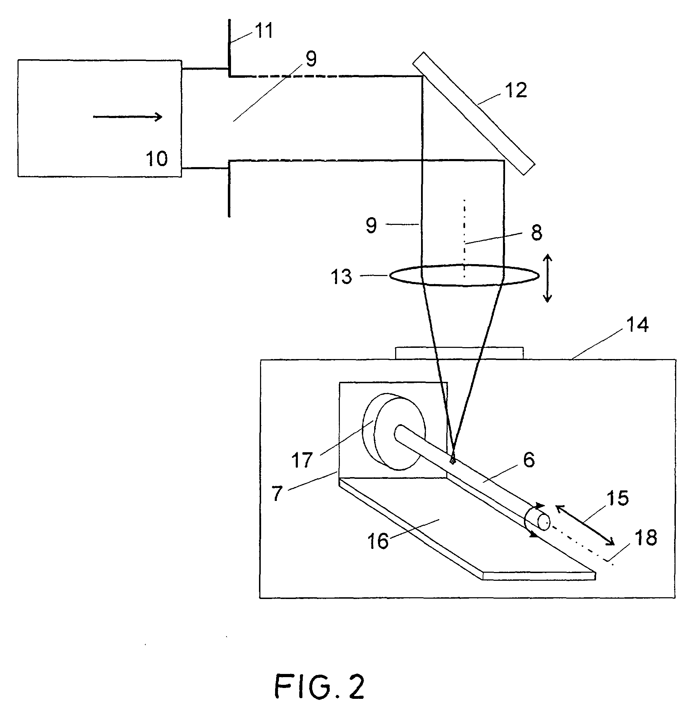

Zur Herstellung des "Stents" 1 mit seiner Struktur aus Längs- und Querstegen 2, 3 sowie den Strängen 4 und Bögen 5 dient die in Fig. 2 gezeigte Vorrichtung. Dabei wird ein zylindrischer PHB-Stentrohling 6 auf einem Manipulator 7 festgelegt, der für eine Relativverschiebung des Rohlings 6 bezüglich des in seiner Strahlachse 8 ortsfesten Laserstrahls 9 sorgt.

Der Laserstrahl 9 wird von einem gepulsten Titan-Saphir-Laser 10 erzeugt und weist eine durchstimmbare Wellenlänge von 760 bis 810 nm auf. Die Pulsenergie beträgt etwa 1 mJ, kann jedoch herunter bis 10 µJ oder weniger gewählt werden. Die Pulsdauer ist variierbar und beträgt mindestens 120 fs. Der Laser arbeitet mit einer Pulswiederhol-Frequenz im Bereich zwischen 0,1 und maximal 10 kHz. Durch entsprechende Ergänzungen des kommerziell erhältlichen Titan-Saphir-Lasers wurde das Lasersystem auf das erfindungsgemäße Verfahren weiter zugeschnitten. So wird ein sogenanntes Lambda/2-Plättchen zur Energievariation und ein schneller mechanischer Shutter zur rechnergesteuerten Ansteuerung des Lasers integriert.

Bezüglich des Strahlengangs des Laserstrahls 9 ist in Fig. 2 angedeutet, daß der Laserstrahl 9 durch eine Blende 11 geführt und unter Zwischenschaltung eines Umlenkspiegels 12 mittels der Linse 13 auf den "Stent"-Rohling 6 abgebildet wird.

Der bereits erwähnte Manipulator 7 ist in einer Vakuumkammer 14 untergebracht. Die Laserbearbeitung fand dabei mit einem Druck von weniger als 10⁻⁴ mbar statt. Im übrigen ist ein Arbeiten unter einem Prozeßgas oder unter Luft ebenfalls möglich.

Der Manipulator 7 weist bezüglich des Werkstückes 6 zwei Achsen auf, nämlich eine lineare Achse 15 in Form eines entsprechend verstellbaren Supports 16. Auf dem Support ist eine Dreheinrichtung 17 mit der Rotationsachse 18 angeordnet, zu der konzentrisch der "Stent"-Rohling 6 in einem nicht näher dargestellten Spannfutter gehalten ist. Durch eine überlagerte Bewegung des Rohlings 6 entlang der linearen Achse 15 und Drehung um die Rotationsachse 18 bei gleichzeitiger Bestrahlung mit ultrakurzen Hochleistungs-Laserimpulsen kann ein "Stent" mit der in Fig. 1 gezeigten Struktur aus dem Rohling 6 exakt und ohne Gratbildung an den Kanten geschnitten werden. Dabei wird aufgrund der Bestrahlungsverhältnisse auch eine thermische oder photochemische Beeinträchtigung des PHB-Materials vermieden, wodurch dessen Materialeigenschaften, wie Resorbierbarkeit und mechanische Elastizität, unverändert bleiben.

Im übrigen ist darauf hinzuweisen, daß der Laserstrahl statt der Abbildung der Blende 11 auch fokussierend auf das Werkstück abgebildet werden kann. Eine weitere Möglichkeit besteht darin, den Laserstrahl mittels einer diffraktiven Optik - was im Fachjargon häufig auch als "Hologramm" bezeichnet wird - auf das Werkstück zu richten. Ferner kann nicht nur mit einer ortsfesten Strahlachse 8 des Laserstrahls 9 gearbeitet werden, sondern dieser - ggf. in Überlagerung mit einer Werkstück-Bewegung - mittels eines Laser-Scanners über das Werkstück geführt werden. Die jeweilige Arbeitsweise wird sich dabei nach der zu erzielenden Struktur und dem Material des Werkstückes richten.

## Patentansprüche

1. Verfahren zur Herstellung feinstrukturierter medizintechnischer Implantate durch Laser-Materialbearbeitung, insbesondere Laser-Schneiden von bioresorbierbaren Gefäßwandstützen (1), wobei die Bearbeitung mit einem gepulsten Laserstahl (9) einer Pulsdauer im Bereich von 10 bis 10000 Femtosekunden und einer angepaßten Kombination der Parameter Pulswiederhol-Frequenz, Puls-Energie und Verfahrgeschwindigkeit des Werkstückes (6) relativ zum Laserstrahl derart erfolgt, daß eine schmelzfreie, materialschonende Bearbeitung des Werkstoffes stattfindet, und wobei ferner ein Laser-Scanner zur Fühnmg des Laserstrahles über das Werkstück (6) eingesetzt wird.

2. Verfahren zur Herstellung feinstrukturierter medizintechnischer Implantate durch Laser-Materialbearbeitung, insbesondere Laser-Schneiden von bioresorbierbaren Gefäßwandstützen (1), wobei die Bearbeitung mit einem gepulsten Laserstahl (9) einer Pulsdauer im Bereich von 10 bis 10000 Femtosekunden und einer angepaßten Kombination der Parameter Pulswiederhol-Frequenz, Puls-Energie und Verfahrgeschwindigkeit des Werkstückes (6) relativ zum Laserstrahl derart erfolgt, daß eine schmelzfreie, materialschonende Bearbeitung des Werkstoffes stattfindet, und wobei ferner eine bioresorbierbare Gefäßwandstütze (1) insbesondere aus Poly-Hydroxy-Buttersäure durch die schneidende Laserbearbeitung mit einer Struktur aus miteinander vernetzten Stegen (2, 3) versehen wird.

3. Verfahren nach Anspruch 1 oder 2, **dadurch gekennzeichnet, daß** die Pulsdauer zwischen 100 und 1000 Femtosekunden beträgt.

4. Verfahren nach einem der Ansprüche 1 bis 3, **dadurch gekennzeichnet, daß** die Pulsenergie etwa 10 µJ bis 1 mJ beträgt.

5. Verfahren nach einem der Ansprüche 1 bis 4, **dadurch gekennzeichnet, daß** die Pulswiederhol-Frequenz zwischen 0,1 und 10 kHz beträgt.

6. Verfahren nach einem der Ansprüche 1 bis 5, **dadurch gekennzeichnet, daß** zur Erzeugung des Laserstrahls ein Titan-Saphir-Laser mit einer durchstimmbaren Wellenlänge von 760 bis 810 nm verwendet wird.

7. Verfahren nach einem der Ansprüche 1 bis 6, **dadurch gekennzeichnet, daß** der Laserstrahl (9) durch eine Blendeneinrichtung (11) geführt und auf das Werkstück (6) abgebildet wird.

8. Verfahren nach einem der Ansprüche 1 bis 6, **dadurch gekennzeichnet, daß** der Laserstrahl (9) auf das Werkstück (6) fokussiert wird.

9. Verfahren nach einem der Ansprüche 1 bis 6, **dadurch gekennzeichnet, daß** der Laserstrahl (9) mittels einer diffraktiven Optik auf das Werkstück (6) gerichtet wird.

10. Verfahren nach einem der Ansprüche 1 bis 9, **dadurch gekennzeichnet, daß** die Laserstrahlbearbeitung in Vakuum (14) oder unter einem Prozeßgas oder in Luft stattfindet.

11. Verfahren nach einem der Ansprüche 1 bis 10, **dadurch gekennzeichnet, daß** das Werkstück (6) zur Laserbearbeitung auf einem Manipulator (7) zur Relativverschiebung bezüglich des in seiner Strahlachse (8) vorzugsweise ortsfesten Laserstrahles (9) geführt wird.

## Claims

1. A method of producing microstructural medical implants by laser material processing, in particular by the laser cutting of bioresorbable vessel wall supports (1), wherein the treatment by a tunable laser beam (9) of a pulse length in the range of 10 to 10000 femtoseconds and an adjusted combination of the parameters of frequency of pulse repetition, pulse energy and of velocity of displacement of the workpiece (6) relative to the laser beam is effected for gentle material treatment without melting to take place, and wherein further a laser scanner is used for the laser beam to be passed over the workpiece (6).

2. A method of producing microstructural medical implants by laser material processing, in particular by the laser cutting of bioresorbable vessel wall supports (1), wherein the treatment by a tunable laser beam (9) of a pulse length in the range of 10 to 10000 femtoseconds and an adjusted combination of the parameters of frequency of pulse repetition, pulse energy and of velocity of displacement of the workpiece (6) relative to the laser beam is effected for gentle material treatment without melting to take place, and wherein further a bioresorbable vessel wall support (1), in particular of poly-hydroxybutyrate, is provided with a structure of cross-linked ribs (2, 3) by cutting lasering.

3. A method according to claim 1 or 2, **characterized in that** the pulse length ranges from 100 to 1000 femtoseconds.

4. A method according to one of claims 1 to 3, **characterized in that** the pulse energy ranges from approximately 10 µJ to 1 mJ.

5. A method according to one of claims 1 to 4, **characterized in that** the frequency of pulse repetition ranges from 0.1 to 10 kHz.

6. A method according to one of claims 1 to 5, **characterized in that** a titanium sapphire laser of a variable wavelength of 760 to 810 nm is used for the production of the laser beam.

7. A method according to one of claims 1 to 6, **characterized in that** the laser beam (9) is led through a diaphragm (11) and projected on the workpiece (6).

8. A method according to one of claims 1 to 6, **characterized in that** the laser beam (9) is focused on the workpiece (6).

9. A method according to one of claims 1 to 6, **characterized in that** the laser beam (9) is directed on to the workpiece (6) by means of a diffractive optic.

10. A method according to one of claims 1 to 9, **characterized in that** the treatment by laser beam takes place in a vacuum (14) or under a processing gas or in the air.

11. A method according to one of claims 1 to 10, **characterized in that** the workpiece (6), for being lasered, is passed on a manipulator (7) by displacement relative to the laser beam (9) which is preferably stationary in its axis (8).

## Revendications

1. Procédé de fabrication d'implants médicaux finement structurés par usinage laser de matière, en particulier découpe laser de soutiens de parois vasculaires biorésorbables (1), l'usinage ayant lieu avec un faisceau laser pulsé (9) d'une durée de pulse comprise entre 10 et 10000 femtosecondes et comprenant une combinaison adaptée des paramètres fréquence de répétition des pulses, énergie pulsée et vitesse de déplacement de la pièce (6) par rapport au faisceau laser de telle façon que l'usinage de la matière soit sans fusion et respectueux de la matière et, de plus, un scanner laser étant utilisé pour guider le faisceau laser au-dessus de la pièce à usiner (6).

2. Procédé de fabrication d'implants médicaux finement structurés par usinage laser de matière, en particulier découpe laser de soutiens de parois vasculaires biorésorbables (1), l'usinage ayant lieu avec un faisceau laser pulsé (9) d'une durée de pulse comprise entre 10 et 10000 femtosecondes et comprenant une combinaison adaptée des paramètres fréquence de répétition des pulses, énergie pulsée et vitesse de déplacement de la pièce (6) par rapport au faisceau laser de telle façon que l'usinage de la matière soit sans fusion et respectueux de la matière et, de plus, un soutien de paroi vasculaire biorésorbable (1), en particulier en acide polyhydroxybutyrique, étant doté, grâce à l'usinage par découpe laser, d'une structure constituée de longerons et d'entretoises (2, 3) formant ensemble un réseau.

3. Procédé selon revendication 1 ou 2, **caractérisé en ce que** la durée de pulse est comprise entre 100 et 1000 femtosecondes.

4. Procédé selon l'une quelconque des revendications 1 à 3, **caractérisé en ce que** l'énergie pulsée est de 10 µJ à 1 mJ environ.

5. Procédé selon l'une quelconque des revendications 1 à 4, **caractérisé en ce que** la fréquence de répétition des pulses est comprise entre 0,1 et 10 kHz.

6. Procédé selon l'une quelconque des revendications 1 à 5, **caractérisé en ce que,** pour produire le faisceau laser, on utilise un laser titane saphir d'une longueur d'onde variable de 760 à 810 mm.

7. Procédé selon l'une quelconque des revendications 1 à 6, **caractérisé en ce que** le faisceau laser (9) passe par un diaphragme (11) et est représenté sur la pièce à usiner (6).

8. Procédé selon l'une quelconque des revendications 1 à 6, **caractérisé en ce que** le faisceau laser (9) est focalisé sur la pièce à usiner (6).

9. Procédé selon l'une quelconque des revendications 1 à 6, **caractérisé en ce que** le faisceau (9) est dirigé sur la pièce à usiner (6) au moyen d'une optique diffractante.

10. Procédé selon l'une quelconque des revendications 1 à 9, **caractérisé en ce que** l'usinage laser se déroule sous vide (14) ou sous un gaz process ou dans l'air.

11. Procédé selon l'une quelconque des revendications 1 à 10, **caractérisé en ce que** la pièce à usiner (6) au laser passe sur un manipulateur (7) pour le déplacement relatif par rapport au faisceau laser (9) de préférence stationnaire sur son axe de faisceau (8).
